(19) Europäisches Patentamt
European Patent Office
Office européen des brevets

(11) Publication number : **0 651 055 A2**

(12) # EUROPEAN PATENT APPLICATION

(21) Application number : **94307882.4**

(22) Date of filing : **26.10.94**

(51) Int. Cl.[6] : **C12N 15/55,** C12N 9/18, A61K 38/46, C12S 13/00, B09C 1/10, A62D 3/00

(30) Priority : **29.10.93 US 145336**

(43) Date of publication of application : **03.05.95 Bulletin 95/18**

(84) Designated Contracting States : **AT BE CH DE DK ES FR GB GR IE IT LI LU MC NL PT SE**

(71) Applicant : **State of Israel Represented by the Prime Minister's Office The Israel Institute for Biological Research
P.O. Box 19
Nes Ziona74100 (IL)**

(72) Inventor : **Shafferman, Avigdor
69 Ben Gurion Street
Ness Ziona 74037 (IL)**
Inventor : **Barak, Dov
20 Usishkin Street
Rehovot 76502 (IL)**
Inventor : **Ordentlich, Arie
36 Hurgin Street
Ramat Gan 52356 (IL)**
Inventor : **Kronman, Chanoch
15 Ha'admor Street
Rehovot 76205 (IL)**
Inventor : **Velan, Baruch
14 Weisel Street
Tel Aviv 64241 (IL)**

(74) Representative : **Hillier, Peter et al
Reginald W. Barker & Co.,
Chancery House,
53-64, Chancery Lane
London, WC2A 1QU (GB)**

(54) **Uses of modified cholinesterase and compositions containing them.**

(57) Detoxification of humans who have been exposed to phosphylating agents, and decontamination of a locus containing phosphylating agents (e.g. organophosphorus insecticides or organophosphorus nerve gases), are effected by administration to the humans or applying to the locus, respectively, at least one recombinant cholinesterase mutant so as to form phosphylated cholinesterase mutant, in presence of intrinsic or extrinsic reactivator activity effective to reactivate the mutant by breaking the phosphyl/cholinesterase mutant covalent bond, the phosphylated mutant being more readily reconverted to nonphosphylated mutant, than the corresponding phosphylated non-mutated cholinesterase is reconverted to non-phosphylated non-mutated cholinesterase. Mutation sites in the amino acid sequence of HuAChE, or in equivalent positions in other cholinesterases, are preferably selected from at least one of (a) Ser203, Glu334, His447 ; (b) Gly122, Tyr133, Glu202, Ser229, Gly448, Glu450 ; (c) Leu76, Tyr77, Trp86, Tyr117, Tyr119, Gly120, Gly121, Trp236, Phe295, Phe297, Tyr337, Phe338, Trp439, Tyr449 ; and/or (d) Tyr72, Asp74, Tyr124, Glu285, Trp286, Tyr341. The invention includes use of the (non-phosphylated) mutants for the manufacture of medicaments for detoxification, pharmaceutical formulations for detoxification, compositions for decontamination, and phosphylated and non-phosphylated recombinant cholinesterase mutants, per se.

EP 0 651 055 A2

## FIELD OF THE INVENTION

The present invention relates to the use of recombinant cholinesterase mutants for detoxification and decontamination of toxic organophosphorus compounds by converting them into phosphylated recombinant cholinesterase mutants, from which the non-phosphylated mutants are regenerated more readily than non-mutated cholinesterases are regenerated from phosphylated non-mutated cholinesterases; and to related compositions, pharmaceutical formulations, and phosphylated and nonphosphylated recombinant cholinesterase mutants.

## BACKGROUND OF THE INVENTION

Cholinesterases (ChE's) are readily phosphylated, at the active site serine, by a variety of organophosphonates and organophosphates. In this connection, the term "phosphyl" in the present specification and claims denotes all tetravalent phosphorus electrophilic groups (c.f. Bourne, N. and Williams, A. (1984) J. Am. Chem. Soc. 1 0 6, 7591-7596) and terms derived therefrom such as "phosphylation" and "phosphylated" have the corresponding meanings as will be understood by persons skilled in the art. The conjugates formed from ChE and organophosphonates or organophosphates (which terms as used herein include the analogs containing 1-3 sulfur atoms instead of the corresponding number of oxygen atoms) then undergo postinhibitory processes, the nature and extent of which depend upon the structure of the inhibitor and the particular enzyme studied. Spontaneous reactivation, through displacement of the phosphyl moiety (Scheme 1) from the active site, is usually very slow; however the enzyme can be reactivated by various oxime nucleophiles, see Aldrich, W.N., and Reiner, E., (1972) Enzyme Inhibitors as Substrates, North-Holland Publishing Co., Amsterdam, and Froede, H.C., and Wilson, I.B., (1971) in: The Enzymes, Vol.5, 3rd Ed. (Boyer, P.D., Ed.), pp 87-114, Academic Press, Orlando FL.

Accumulation of acetylcholine at cholinergic receptor sites as a result of AChE inhibition due to phosphylation, produces effects equivalent to excessive stimulation of these receptors, throughout the central and peripheral nervous system. The cholinergic crisis thus produced is characterized by miosis, increased salivary and tracheobronchial secretions, bradycardia, muscle weakness, fasiculations, and convulsions, resulting in death by respiratory failure.

It has previously been proposed to treat poisoning due to toxic phosphylating compounds, with a method consisting of pretreatment with a reversible ChE inhibitor, followed by treatment with a combination of atropine (to counteract the effect of accumulated acetylcholine) and reactivator, to reactivate phosphylated AChE. This method is effective to prevent death in experimental animals but is ineffective in protecting against performance deficits, convulsions or permanent brain damage. It has further been proposed to use enzymes such as ChE's as single pretreatment drugs to sequester highly toxic phosphylating agents before they reach their cholinesterase physiological targets (see e.g. Wolfe, A.D., et al., (1987) Fundam. Appl. Toxicol. 9: 266; Ashani, Y., et al., (1991) Biochem. Pharmacol. 41: 37; and Bloomfield, C.A., et al., (1991) J. Pharmacol. Exp. Therap. 259: 633. The use of ChE's in this manner has the disadvantage that because of the phenomenon of aging (see below), a relatively large quantity of these enzymes may be necessary, which in turn may also have undesirable effects on the body. Moreover, reactivation of phosphylated ChE's, e.g. by use of oxime reactivators as mentioned above, may be thwarted if aging is relatively fast as compared with the rate of reactivation, as appears to be generally the case when dealing with phosphylated natural cholinesterases.

It has been suggested that aging is a unimolecular process of dealkylation through alkyl-oxygen bond scission resulting in a formal negative charge in the organophosphonate or organophosphate-ChE conjugate (see Scheme 1, which is merely illustrative and not exhaustive of the possible phosphorus containing reactants which may be involved), see Michel, H.O., et al., (1967) Arch. Biochem. Biophys. 121, 29-34, and Berman, H.A. and Decker, M.M. (1986) Biochim. Biophys. Acta 872, 125-133. The aged organophosphonate and organophosphate-conjugates are refractive to reactivation and thus treatment, following intoxication with certain organophosphonate and organophosphate insecticides or nerve gas agents, is made extremely difficult.

Scheme 1:

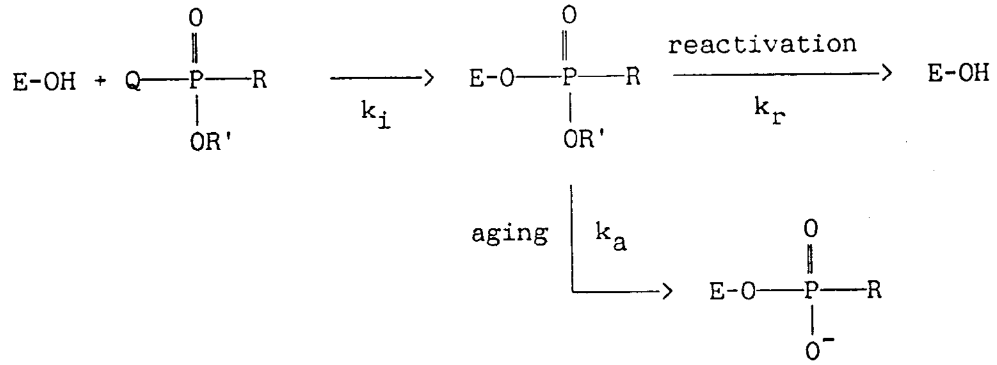


In Scheme 1, E is the enzyme residue which includes an -OH susceptible to phosphylation, and, in general, Q is a leaving group, R and R' are the same or different monovalent hydrocarbyl residues, or R is may be a monovalent hydrocarbyloxy residue. Alternatively, any one or more of the oxygen atoms depicted may of course be replaced by sulfur atoms.

Recently, there have been generated a large number of recombinant human acetylcholinesterase (HuAChE) mutants; amino acids important for maintaining the structural integrity of the enzyme (Velan, B., et al., (1991) J. Biol. Chem. 266, 23977-23984, and Shafferman, A., et al., (1992) J. Biol. Chem. 267, 17649-17648), as well as residues involved in catalysis and in interactions with various substrates and reversible inhibitors (Shafferman, A., et al., (1992) in: Multidisciplinary Approaches to Cholinesterase Functions (Shafferman A. and Velan B.. Eds), pp.165-175, Plenum Publishing Corporation, New York; Shafferman, A., et al., (1992) EMBO J. 11, 3561-3568; and Ordentlich, A., et al., (1993) J. Biol. Chem. 268, 17 0 83-17 0 95), have been identified.

A limited number of phosphylated cholinesterase mutants are known. These are Glu199Gln TcAChE and Glu199Asp TcAChE which have been phosphylated by reaction with haloxon, paraoxon, DFP and MEPQ, see Radic, Z., et al., (1992) Biochemistry 31, 976 0 -9767; and the mouse AChE mutants Phe295Leu, Phe297Ile, Phe295Leu/Phe297Ile, Arg296Ser and Val3 00 Gly which have been phosphylated with $_2$(Me$_2$CHNH) (O=)P-O-P(=O) (NHCHMe$_2$)$_2$ (iso-ompa), see Vellom, D.C., et al, (1993) Biochemistry, 32: 12. To the best of the present inventors' knowledge, it has not been suggested that the known phosphylated cholinesterase mutants would be more readily reconverted to the non-phosphylated mutants than phosphylated cholinesterases are reconverted to nonphosphylated cholinesterases.

It is an object of the present invention to use particular recombinant cholinesterase mutants for the purpose of detoxifying or decontaminating phosphylating agents such as organophosphorus insecticides or nerve gases by converting them into more readily reactivated phosphylated recombinant human acetylcholinesterase mutants. Other objects of the invention will appear from the description which follows.

## SUMMARY OF THE INVENTION

In one aspect, the present invention provides a method for detoxification of humans who have been exposed to a toxic phosphylating agent, which comprises administration to the humans at least one recombinant cholinesterase mutant so as to form phosphylated cholinesterase mutant, in presence of reactivator activity effective to reactivate the mutant by breaking the phosphyl/cholinesterase mutant covalent bond, the phosphylated mutant being characterized by the fact that it is more readily reconverted to non-phosphylated mutant, than the corresponding phosphylated non-mutated cholinesterase is reconverted to nonphosphylated non-mutated cholinesterase.

In another aspect, the invention provides a method for decontamination of a locus containing a toxic phosphylating agent, which comprises applying to the locus at least one recombinant cholinesterase mutant so as to form phosphylated cholinesterase mutant, in presence of reactivator activity effective to reactivate the mutant by breaking the phosphyl/cholinesterase mutant covalent bond, the phosphylated mutant being characterized by the fact that it is more readily reconverted to non-phosphylated mutant, than the corresponding phosphylated non-mutated cholinesterase is reconverted to nonphosphylated non-mutated cholinesterase.

In still another aspect, the invention provides a recombinant cholinesterase mutant which is characterized by the facts that:

(i) a phosphylated product thereof is more readily reconverted to non-phosphylated mutant, than the corresponding phosphylated non-mutated cholinesterase is reconverted to non-phosphylated non-mutated cholinesterase; and

(ii) it comprises intrinsic reactivator activity effective to reactivate the mutant from a phosphylated product thereof, by breaking the phosphyl/cholinesterase mutant covalent bond.

The present invention moreover proyides a pharmaceutical formulation useful for detoxification of humans who have been exposed to a toxic phosphylating agent, as well as a composition useful for decontamination of a locus containing a toxic phosphylating agent, each of which comprises a recombinant cholinesterase mutant characterized by the fact that a phosphylated mutant thereof is more readily reconverted to nonphosphylated mutant, than the corresponding phosphylated non-mutated cholinesterase is reconverted to non-phosphylated non-mutated cholinesterase; and which comprises also at least one discrete reactivator compound effective to reactivate the mutant from a phosphylated product thereof, by breaking the phosphyl/cholinesterase mutant covalent bond, and at least one non-active ingredient, which in the case of the decontaminating composition is selected from diluents, carriers and surface active agents, and in the case of the pharmaceutical formulation is selected from pharmaceutically acceptable carriers, diluents and adjuvants.

The present invention provides in a further aspect, a phosphylated reaction product of a recombinant cholinesterase mutant with a tetravalent phosphorus electrophilic group, such phosphylated reaction product being characterized by the fact that it is more readily reconverted to non-phosphylated mutant, than the corresponding phosphylated non-mutated cholinesterase is reconverted to non-phosphylated non-mutated cholinesterase; provided that said phosphylated reaction product does not include the following categories (i) and (ii):

(i) Glu199Gln TcAChE and Glu199Asp TcAChE which have been phosphylated by reaction with a phosphylating agent selected from the group consisting of haloxon, paraoxon, DFP and MEPQ;

(ii) mouse AChE mutants Phe295Leu, Phe297Ile, Phe295Leu/Phe297Ile, Arg296Ser and Val3 00 Gly which have been phosphylated by reaction with $_2(Me_2CHNH)$ (O=)P-O-P(=O) $(NHCHMe_2)_2$.

BRIEF DESCRIPTION OF THE DRAWING

Figure 1 shows the results of active site titration of HuAChE and its mutants with soman and MEPQ.

DETAILED DESCRIPTION OF THE INVENTION

It may be noted, in general, that where in the present specification and claims, a number denoting an amino acid position in the enzyme structure is preceded by a symbol for an amino acid, this identifies the amino acid in that position in the unmutated recombinant enzyme; where the number is followed additionally by a symbol for an amino acid, this identifies the amino acid in that position in the mutated recombinant enzyme.

It will be apparent from what has been stated above, that the present invention is concerned with cholinesterase mutants, and more particularly their use in detoxification and decontamination. Persons in the field will be aware that there exists substantial conservation and homology between different cholinesterases, whatever their sources. Thus, the amino acid sequence is similar in the different cholinesterases, see e.g. Krejci, E. et al., (1991) P.N.A.S. 88: 6647. Replacement of amino acids in corresponding sites in the "gorge" in different cholinesterases would therefore lead to products having similar chemical characteristics and catalytic activity (see e.g., for torpedo acetylcholinesterase (TcAChE), Radic, Z., et al., loc cit; and Harel N., et al.,(1992) P.N.A.S. 89: 1 0 827; for mouse AChE, Vellom, D.C., et al., loc cit; for human butyrylcholinesterase, Neville, L.F., et al., (199 0 ) J. Biol. Chem., 265: 2 0 735; for Drosophila AChE, Fournier, D., et al., (1992) J. Biol. Chem., 267: 1427 0 ).

Because of the analogies referred to in the preceding paragraph, between the different cholinesterases, persons who are skilled in the art will be aware of the meaning of the phrase "equivalent positions in other cholinesterases", as used in the present specification and claims, in relation to particular amino acid positions in the amino acid sequence of human acetylcholinesterase. In other words, a person of the art who is made aware of the present invention, in particular as it concerns mutants at particular amino acid sites in human acetylcholinesterase, would have no difficulty in applying the invention to corresponding amino acid sites in other cholinesterases.

The particular amino acid sites in the amino acid sequence in human acetylcholinesterase, at one or more of which sites mutation is effected for the purposes of particular embodiments of the present invention, since it is presently believed that mutation at one or more of these sites will have the desired effect described elsewhere in relation to breaking the phosphyl/cholinesterase mutant bond, may be arranged according to subgroups (a), (b), (c) and (d), which will be set forth and explained below. In order to emphasize the homology referred to above, the corresponding amino acid sites in parentheses refer to the position of analogous residues in Torpedo AChE (TcAChE) according to the recommended nomenclature (see Massoulie, J., et al., at pp.285-288, in (1992) Multidisciplinary Approaches to Cholinesterase Functions, Shafferman A. and Velan B., Eds., Plenum Publishing Corporation, New York). These sub-groups (a), (b), (c) and (d) are as follows:

(a) Ser203* (200), Glu334 (327), His447 (440);

(b) Gly122 (119), Tyr133 (130), Glu202 (199), Ser229 (226), Gly448 (440), Glu450 (443);

(c) Leu76 (74), Tyr77 (75), Trp86 (84), Tyr117 (114), Tyr119 (116); Gly120 (118), Gly121 (119), Trp236 (233), Phe295 (288), Phe297 (290), Tyr337 (Phe330), Phe338 (331), Trp439 (432), Tyr449(442);

(d) Tyr72 (70), Asp74 (72), Tyr124 (122), Glu285 (278), Trp286 (279), Tyr341 (334).

---

*example of a mutation is e.g. Ser203Cys, where the SH may be phosphylated instead of the OH in Ser203 when not mutated.

Sub-groups of amino acids (a), (b) and (c), above, or their equivalents as discussed above, constitute the active center in cholinesterases in general and in HuAChE (and TcAChE) in particular, of which sub-group (a) is the catalytic triad (Shafferman, A., et al., (1992) J. Biol. Chem., loc cit), subgroup (b) is the hydrogen bond network (Ordentlich, A., et al., (1993) FEBS Lett., in the press), and sub-group (c) describes the amino acids lining the active center gorge (Barak, D., et al., (1992) in: Multidisciplinary Approaches to Cholinesterase Functions (Shafferman A. and Velan B.. Eds), pp.195-188, Plenum Publishing Corporation, New York; Shafferman, A., et al., (1992) in: Multidisciplinary Approaches to Cholinesterase Functions, loc cit. pp.165-175; Shafferman, A., et al., (1992) EMBO J., loc cit; and Ordentlich, A., et al., (1993) J. Biol. Chem. loc cit). Sub-group of amino acids (d), or their equivalents as discussed above, constitute the peripheral anionic site, where the amino acid sites in question are nevertheless believed to have an effect on the structure of the active center, see Shafferman, A., et al., (1992) EMBO J., loc cit; Ordentlich, A., et al., (1993) J. Biol. Chem. loc cit; and Barak, D., et al., (1993) J. Biol. Chem., in the press.

As regards the phosphylated reaction products of recombinant cholinesterase mutants with tetravalent phosphorus electrophilic groups to which the invention relates in part, and which are in any event formed in the present decontamination and detoxification methods, such reaction products are characterized by the fact that they are more readily reconverted to nonphosphylated mutant, than the corresponding phosphylated non-mutated cholinesterase is reconverted to non-phosphylated non-mutated cholinesterase. Thus, in the present case, the phosphylation products are less likely to be converted to refractory aged products which cannot be reconverted to the prephosphylated enzyme. The consequence of this situation is that in presence of reactivator activity, a small quantity of enzyme can be reused in situ an infinite number of times, instead of being locked into an aged non-regenerable product. To express in another manner the characteristic that the phosphylated mutant is more readily reconverted to non-phosphylated mutant, than in the case of the non-mutated analogs, this includes the concept that in the phosphylated mutants to which the present invention relates, the rate of aging is less than in the case of the analogous phosphylated non-mutated enzymes.

As previously stated, in one embodiment of the invention the necessary reactivator activity for reconverting phosphylated enzyme to pre-phosphylated enzyme is intrinsic, i.e. it lies within the enzyme itself. Intrinsic reactivation ability is observed in certain phosphylated cholinesterases, and it is contemplated that this ability can be reinforced by appropriate mutation of amino acids, which may affect the structure of the active center.

In another embodiment of the invention, the necessary reactivator activity is provided by at least one discrete reactivator compound. Non-limiting examples of such reactivator compounds are obidoxime chloride (Merck Index, 1 0 th Edn., 1983, monograph no. 6581); pralidoxime chloride, iodide and mesylate (Merck Index, 1 0 th Edn., 1983, monographs nos. 7599-76 0 1;) and HI-6 (Roussaux, C.G. and Dua, A.K. (1989) Can. J. Physiol. Pharmacol. 67, 1183-1189).

Phosphylating agents (or, otherwise expressed, agents which give rise to tetravalent phosphorus electrophilic groups) which in effect may be scavenged in accordance with the decontamination and detoxification methods of the invention, and which form phosphylated cholinesterases in accordance with a particular aspect of the invention, are well known in the art. As previously pointed out, they may be organophosphates (e.g. DFP, paroxon) or organophosphonates (e.g. sarin, soman), their sulfur-containing analogs (e.g. parathion, fenthion, dimpylate) analogs, or similar compounds containing an amide group (e.g. tabun).

Without prejudice to the generality of the invention in this respect, the tetravalent phosphorus electrophilic group may be derived from a compound of the formula

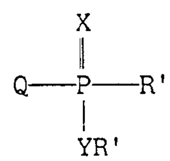

wherein Q is a leaving group, R' is R" or ZR", each of R' and R", which may be the same as or different from each other, is selected from the group consisting of $C_1$-$C_{10}$ alkyl, $C_3$-$C_{10}$ cycloalkyl and $C_3$-$C_{10}$ cycloalkyl substituted by up to two $C_1$-$C_4$ alkyl groups, and X, Y and Z are each independently O or S.

The leaving group Q may be for example halogen such as fluorine or chlorine, cyanide, thiocyanate, $C_{1-6}$ alkylthio, carbocyclic arylthio such as phenylthio (which may be substituted by one or more $C_{1-4}$ alkyl, $C_{1-4}$ alkoxy, $C_{1-4}$ alkylthio or nitro groups), carbocyclic aryloxy such as phenoxy (which may be substituted by one or more $C_{1-4}$ alkyl, $C_{1-4}$ alkoxy, $C_{1-4}$ alkylthio or nitro groups), heterocyclic arylthio such as pyridinylthio, quinolinylthio, pyrimidinylthio, pyrazinylthio or pyridazinylthio (any of which may be substituted by one or more $C_{1-4}$ alkyl, $C_{1-4}$ alkoxy, $C_{1-4}$ alkylthio or nitro groups), or heterocyclic aryloxy such as pyridinyloxy, quinolinyloxy, pyrimidinyloxy, pyrazinyloxy, or pyridazinyloxy (any of which may be substituted by one or more $C_{1-4}$ alkyl, $C_{1-4}$ alkoxy, $C_{1-4}$ alkylthio or nitro groups).

Alkyl groups, whether present in their own right or as part of a larger group such as alkoxy or alkylthio may be (subject to the stated limitations as to the number of carbon atoms), e.g., methyl, ethyl, propyl, isopropyl, n-butyl, isobutyl, sec-butyl, tert-butyl, pentyl, hexyl, pinacolyl, heptyl, octyl, 2-ethylhexyl, nonyl or decyl. $C_3$-$C_{10}$ cycloalkyl may be e.g. cyclopropyl, cyclobutyl, cyclopentyl, cyclohexyl, cycloheptyl, cyclooctyl, cyclononyl or cyclodecyl. $C_3$-$C_{10}$ cycloalkyl substituted by up to two $C_1$-$C_4$ alkyl groups may be e.g. 2-methylcyclopropyl, 2,2-dimethylcyclopropyl, 2-methylcyclobutyl, 2,2-dimethylcyclobutyl, 3-methylcyclopentyl, 4-methylcyclohexyl, 4,4-dimethylcyclohexyl, correspondingly substituted cycloheptyl, cyclooctyl, cyclononyl and cyclodecyl groups, and analogs of such groups substituted by e.g. ethyl instead of methyl. Non-limiting examples of compounds of the above formula are as follows:

| Name | Q | X | R' | YR' |
|---|---|---|---|---|
| DFP | F | O | i-PrO | I-PrO |
| paroxon | p-nitrophenoxy | O | EtO | EtO |
| sarin | F | O | Me | i-PrO |
| soman | F | O | Me | pinacolyl-O |
| MEPQ | 7-(1-methyl)quinolinium | O | Me | EtO |
| parathion | p-nitrophenoxy | S | EtO | EtO |
| fenthion | 3-methyl-4-methylthiophenoxy | S | MeO | MeO |
| dimpylate | 2-isopropyl-6-methyl-4-pyrimidinyl | S | EtO | EtO |

The method for decontaminating a locus containing phosphylating agents in accordance with the invention, may be carried into effect for example by use of a composition which may contain the usual non-active ingredients such as diluents, carriers and surface active agents, besides of course an active decontaminating agent

which is one or more of recombinant cholinesterase mutants as defined herein, and reactivator activity.

The method for detoxification of humans who have been exposed to phosphylating agents by administration to the humans of recombinant cholinesterase mutants in accordance with the invention may be carried out, for example by administering to the human a pharmaceutical formulation comprising at least one of the recombinant cholinesterase mutants as defined herein, and reactivator activity. The preparation of pharmaceutical formulations, e.g. for oral, parenteral, percutaneous, rectal or topical administration is of course well known in the pharmaceutical art. It may be mentioned, however, that such formulations may comprise in addition to the mutant and reactivator activity, at least one non-active ingredient selected from pharmaceutically carriers, diluents and adjuvants.

As regards mutation at the preferred amino acid sites, this may be carried out as indicated below. It will be appreciated that the following description is only exemplary and non-limiting, since persons skilled in the art will readily be able to suggest alternative procedures, which are of course all within the contemplation of the present invention. Cholinesterases other than HuAChE used in the present exemplification may of course be mutated in similar fashion. Mutation at more than one mutation site will also be within the capability of the skilled person on the basis of information in the present specification, as well as in the relevant scientific literature.

Mutagenesis of rHuAChE may be performed by cassette replacement into a series of HuAChE sequence variants which conserve the wild-type coding specificities but carry new unique restriction sites as outlined in Shafferman, A. et al., (1992) J. Biol. Chem., loc cit.

Tyr72, Asp74, Leu76, Tyr77, and Trp86 may be mutated by replacing the AccI-NruI DNA fragment of the Ew4 AChE DNA derivative with the appropriate synthetic DNA duplexes as demonstrated in Shafferman, A., et al., (1992) EMBO J., loc cit.

Tyr117, Tyr119, Gly120, Gly121, Gly122, and Tyr124 may be mutated by replacing the NruI-NarI DNA fragment of the Ew4 AChE DNA derivative with the appropriate synthetic DNA duplexes as demonstrated in Shafferman, A., et al., (1992) J. Biol. Chem., loc cit.

Tyr133 be mutated by replacing the NarI-XhoI DNA fragment of the Ew4 AChE DNA derivative with the appropriate synthetic DNA duplexes as demonstrated in Shafferman, A., et al., (1992) J. Biol. Chem., loc cit.

Glu202 and Ser203 may be mutated by replacing the BstEII-SphI DNA fragment of native HuAChE cDNA, as outlined in Shafferman, A., et al., (1992) J. Biol. Chem., loc cit..

Ser229 and Trp236 may be mutated by replacing the BstEII-SalI fragment of the Ew7 derivative, as outlined in Shafferman, A., et al., (1992) J. Biol. Chem., loc cit.

Glu285, Trp286, Phe295 and Phe297 may be mutated by replacing the MluI-NarI DNA fragment of the Ew7 derivative with the appropriate synthetic DNA duplexes as demonstrated in Ordentlich, A. et al., (1993) J. Biol. Chem., loc cit.

Glu334, Tyr337, Phe338 and Tyr341 may be mutated by replacing the BclI-NarI DNA fragment of the Ew5 derivative with the appropriate synthetic DNA duplexes as demonstrated in Shafferman, A., et al., (1992) EMBO J., loc cit.

Trp439, His447, Gly448, Trp449 and Glu450 may be mutated by replacing the BstBI-BamHI fragment of the Ew3 derivative with the appropriate synthetic DNA duplexes, as demonstrated in Shafferman, A., et al., (1992) J. Biol. Chem., loc cit.

## DESCRIPTION OF THE PREFERRED EMBODIMENTS

### Starting materials

Acetylthiocholine iodide (ATC), 5:5 -dithiobis (2-nitrobenzoic acid) (DTNB), diisopropyl phosphorofluoridate (DFP) were purchased from Sigma. S-3,3 -dimethylbutyl thioacetate ($CH_3C(=O)$-S-$(CH_2)_2C(CH3)3$ - TB) was synthesized and purified as described previously (Ordentlich, A. et al., (1993) J. Biol. Chem., loc cit). For the preparation of 7-(O-ethyl methylphosphonyloxy)-1-methylquinolinium iodide (MEPQ), see Levy, D. and Ashani, Y. (1986) Biochem. Pharmacol. 35, 1079-1085).

### Recombinant HuAChE and its mutants

Expression of recombinant HuAChE and its mutants in a human embryonal kidney derived 293 cell line, was described previously (see Shafferman, A. et al., (1992) J. Biol. Chem., loc cit; Velan, B., et al., (1991) Cell. Mol. Neurobiol. 11, 143-156; and Kronman, C., et al., (1992) Gene 121, 295-304). Generation of mutants Glu202Gln(Glu199) and Tyr337Ala(Phe330) was described previously (Ordentlich, A. et al., (1993) J. Biol. Chem., loc cit). Substitution of residue Glu450 (Glu443) was carried out by replacement of the BstBI-BamHI

DNA fragment of the AChE-w3 variant (Shafferman, A. et al., (1992) J. Biol. Chem., loc cit) with synthetic DNA duplexes carrying the GCC(Ala) codon. Stable recombinant cell clones expressing high levels of each of the mutants were established according to the procedure described previously (Kronman, C., et al., loc cit). The resulting recombinant clones were propagated in multitray systems (Lazar, A., et al., (1993) Cytotechnology, in the press). The secreted enzymes in the cell supernatant (2-6 liters) were purified (over 90 % purity) by affinity chromatography as described previously (Kronman, C., et al., loc cit).

Kinetic study and analysis of data - AChE activity

This was carried out in the presence of 0.1 mg/ml BSA, 0.3mM DTNB 50 mM sodium-phosphate buffer pH-8.0 ) at 27°C and monitored by a Thermomax microplate reader (Molecular Devices), see Ellman, G.L., et al., (1961) Biochem. Pharmacol. 7, 88-95. Michaelis-Menten constant (Km) values were obtained from the double reciprocal Lineweaver Burk plots and $k_{cat}$ calculations were based on ELISA quantitations (Shafferman, A., et al., (1992) EMBO J., loc cit), as well as on determination of active site concentration by MEPQ titrations (Velan B., et al., loc cit).

Results of active site titration of HuAChE and its mutants with soman and MEPQ are shown in Fig. 1. In all titration experiments the concentration of the catalytic subunits of HuAChE and its mutants was approximately 0.12 µg/ml as determined by ELISA. The residual activity was determined after 30 and 60 minutes incubation (27°C) with soman (wild type HuAChE (solid squares), Tyr337Ala HuAChE (solid circles), Glu202Gln HuAChE (solid triangles) and Glu450Ala HuAChE (+) ) and MEPQ (wild type HuAChE (hollow squares), Tyr337Ala HuAChE (hollow circles), Glu202Gln HuAChE (hollow triangles) and Glu450Ala HuAChE (x) ). The intercepts for zero activity were determined by extrapolation and the values of active site concentration were approximately $2.05x10^{-12}$ moles/ml for MEPQ and twice as high for soman titration.

Phosphylation of recombinant HuAChE mutants

Phosphylation experiments were carried out in at least 4 different concentrations of organophosphonate or organophosphate-inhibitor (I) and two concentrations of enzyme, and enzyme residual activity (E) at various times was monitored. The apparent bimolecular phosphylation rate constants ($k_i$ scheme 1) determined under pseudo-first order conditions were computed from the plot of slopes of ln(E) vs time at different inhibitor concentrations. Rate constants under second order conditions were determined from plots of $\ln\{E/[I_o-(E_o-E)]\}$ versus time. In aging experiments the initial organophosphonate or organophosphate-conjugates were obtained under conditions where $k_i[Io] \gg k_a$ (see scheme 1) and with over 98% inhibition of the initial enzyme activity. The reactivatable (nonaged organophosphonate or organophosphate-conjugate) fraction was determined by reactivation with HI-6 under conditions where $k_r > k_a$ (scheme 1). The excess organophosphonate or organophosphate-inhibitor was removed either by column filtration (Sephadex, G15) or by 100 fold dilution, prior to reactivation. The activity of the reactivated enzyme (Er) was routinely corrected for the inhibitory effect of the reactivator (De Jong, L.P.A. and Kossen, S.P. (1985) Biochim. Biophys. Acta 830, 345-348). The first order rate constants of aging $k_a$ were determined from the slopes of ln(Er) vs. time.

Effect of mutation on hydrolysis of charged and uncharged substrates

Without prejudice to the generality of the recombinant cholinesterase mutants which may be used for purposes of the invention, the four HuAChE mutants Phe338Ala, Tyr337Ala, Glu202Gln and Glu450Ala were compared with respect to their efficiency in catalyzing the hydrolysis of acetylthiocholine (ATC) and its uncharged isostere S-3,3-dimethylbutyl thioacetate (TB). As shown previously (Ordentlich, A., et al., (1993) J. Biol. Chem., loc cit), replacement of the aromatic residue Tyr337 had only a marginal effect on the kinetic parameters ($K_m$ and $k_{cat}$) for the two substrates (Table 1) while the corresponding values for hydrolysis of ATC catalyzed by Glu202Gln HuAChE (Shafferman, A., et al., (1992) in: Multidisciplinary Approaches to Cholinesterase Functions, loc cit) or of the corresponding mutation Glu199Gln in TcAChE (Radic, Z., et al., loc cit) differ significantly from those of the wild type enzyme. It is now demonstrated that this decrease in catalytic efficiency of Glu202Gln is observed also for the noncharged substrate TB. This indicates that the effect of replacement of Glu202 of HuAChE on catalysis is not solely due to electrostatic interactions with the substrate. A similar pattern of reduction of the catalytic parameters, for both ATC and TB, is observed when residue Glu450, which is remote from the active site, is replaced by Ala. Corresponding data for Phe338Ala show a similarity to Phe337Ala when using ATC, but a similarity to Glu202Gln when using TB. Results for Tyr133Ala (using ATC) show the most pronounced difference from wild type HuAChE of the tabulated mutants (see Table 1).

Table 1: Kinetic constants for acetylthiocholine iodide (ATC) and S-3,3-dimethylbutyl thioacetate (TB) by HuAChE and its mutants.

| HuAChEs | ATC | | | TB | | |
|---|---|---|---|---|---|---|
| | $K_m$(mM) | $k_{cat}$* | $k_{app}$♦ | $K_m$(mM) | $k_{cat}$* | $k_{app}$♦ |
| wild type | 0.13±0.015 | 3.70±0.3 | 28.5 | 0.28±0.08 | 0.5 ±0.1 | 1.8 |
| Phe338Ala | 0.24±0.02 | 2.40±0.3 | 10.0 | 0.30±0.07 | 0.13±0.04 | 0.43 |
| Tyr337Ala | 0.10±0.01 | 1.00±0.1 | 10.0 | 0.20±0.03 | 0.2 ±0.05 | 1.0 |
| Glu202Gln | 0.35±0.04 | 0.75±0.15 | 2.1 | 0.23±0.03 | 0.1 ±0.04 | 0.43 |
| Glu450Ala | 0.35±0.04 | 0.10±0.02 | 0.3 | 0.20±0.04 | 0.006±0.001 | 0.03 |
| Tyr133Ala | 5.75 | 0.40 | 0.07 | | | |

*(x $10^{-5}$.$min^{-1}$)
♦(x $10^{-8}$.$M^{-1}$.$min^{-1}$); apparent bimolecular rate constant ($k_{app}$) = $k_{cat}/K_M$

Interaction of HuAChE mutants with organophosphonate or organophosphate inhibitors

To determine the effects of mutations on the stoichiometry of the inhibition reaction of the various AChEs by the organophosphonate or organophosphate inhibitors, the recombinant enzymes were titrated with different concentrations of soman and MEPQ. The organophosphonate MEPQ is a potent inhibitor of AChEs, commonly used as titrant for standardization of concentration of AChE active sites (Levy, D., et al., loc cit). As shown in Fig. 1, the wild type enzyme and all the mutants examined show the expected 1:1 stoichiometry in reactions with MEPQ. The concentrations of the active site subunits, determined by this method, are in good agreement (±10 %) with results obtained from the quantitative immunological (ELISA) assays. Furthermore, under similar experimental conditions, the titration studies demonstrate that for soman 1:2 stoichiometry is obtained for the wild type enzyme as well as for the mutants examined. Due to the chirality of the phosphorous in soman, this organophosphonate or organophosphate-inhibitor is known to exhibit a marked stereoselectivity in reactions with AChE from various sources (Benschop, H.P. and De Jong, L.P.A. (1988) Acc. Chem. Res. 21, 368-374), including recombinant HuAChE (Velan, B., et al., loc cit). Thus, the HuAChE mutants studied appear to retain the stereoselectivity towards soman diastereomers. It is noted, however, that for some mutants, e.g. Phe295Ala or Phe297Ala, stereoselectivity and substrate selectivity may change (Barak, D., et al., loc cit; Ordentlich, A., et al., (1993) J. Biol. Chem., loc cit). The actual bimolecular reaction rates of phosphylation of HuAChE and its mutants, by DFP and soman, were determined under either pseudo first order or second order conditions (Table 2). For the wild type enzyme the rates obtained for DFP and soman are in agreement with values published previously for different AChEs (Gray, P.J. and Dawson, R.M. (1987) Toxicol. Appl. Pharmacol. 91, 140-144; Benschop, H.P., et al., (1984) Toxicol. Appl. Pharmacol. 72, 61-74; and Anderson, R.A., et al., (1977) Gen. Pharmacol. 8, 331-334). It appears that replacement of Tyr337 or Phe338 by alanine had a minimal effect on the bimolecular rate constants of reaction with soman or DFP, but a greater effect with respect to reaction with paraoxon. On the other hand, replacement of Glu202 by glutamine resulted in a marked decrease of the phosphylation rates. Radic Z., et al., loc cit, have recently reported similar effects for the reactivity of an analogous mutant of TcAChE (Glul99Gln) with DFP. Again, as for the acylation process, we find that the effect of replacement of Glu450 on the rates of phosphylation (Table 2), parallels that of Glu202 substitution. It is worth noting that despite the marked decrease in the rates of phosphylation of the HuAChE Glu202Gln and Glu450Ala mutants, the relative reactivity of DFP and soman with each mutant resemble that observed for the wild type enzyme.

Examination of the process of aging through measurements of the enzyme residual activity (De Jong,

L.P.A. and Kossen, S.P. (1985) Biochim. Biophys. Acta 830, 345-348) was carried out with the potent oxime reactivator HI-6 (Roussaux, C.G. et al., loc cit). Under the experimental conditions used, substantial regeneration of enzymatic activity was achieved, even for the pinacolyl methylphosphonyl derivative of the wild type HuAChE which proceeds very rapidly towards the non-reactivatable (aged) enzyme. It appears that reactivation of organophosphonate or organophosphate conjugates with the wild type HuAChE and Tyr337Ala enzymes proceeds at comparable rates while for the Glu202Gln and Glu450Ala mutants, reactivation is slower (data not shown). The first order rate constant for aging of the diisopropyl phosphoryl derivative of HuAChE was determined to be $3.5.10^{-4}$ $min^{-1}$. This value is 260 fold lower than that for the corresponding pinacolyl methylphosphonyl derivative (Table 2), and in good agreement with values published for the same organophosphonate or organophosphate-conjugates in other AChEs (De Jong, L.P.A., et al., loc cit; and Benschop, H.P., et al., loc cit). For the diisopropyl phosphoryl derivatives of Glu202Gln and Glu450Ala HuAChE s the rates of aging appear to be very slow and not measurable under the experimental conditions used. Consequently, there is no experimental evidence that the aging process occurs in these cases. For the pinacolyl methylphosphonyl derivative of the Tyr337Ala, the first order rate constant for aging is similar to that of the wild type enzyme while the corresponding values for Phe338Ala, Glu202Gln and Glu450Ala are approximately 75, 150 and 25 fold lower respectively. Assuming a similar decrease in the rate of aging for the diisopropyl phosphoryl derivatives of Glu202Gln and Glu450Ala, then one could expect a half life of 83 and 17 days respectively, for these processes. It is therefore not surprising that no aging could be observed with DFP - conjugates of these mutants.

Table 2: Rate constants of phosphylation ($k_i$) by diisopropyl phosphorofluoridate (DFP) and pinacolyl methylphosphonofluoridate (soman) of HuAChE and its mutants and aging ($k_a$) of the phosphylated products.

| HuAChEs | $k_i$♥ | | | $k_a$§ |
|---|---|---|---|---|
| | DFP | soman** | paraoxon | soman*** |
| wild type | 9.6 ±0.52(1.0) | 8600±1970(1.0) | 97 ±16 (1.00) | 91 ±12 (1.0) |
| Phe338Ala | 2.8 ±0.15(0.27) | 2900±3000(1.0) | 7.1±0.6 (0.07) | 180 ±64 (2.0) |
| Tyr337Ala | 3.1 ±0.15(0.32) | 2950± 640(0.34) | 14.4±1.4 (0.15) | 180 ±64 (2.0) |
| Glu202Gln | 0.18 ±0.06(0.019) | 310± 100(0.036) | 2.8±0.48(0.03) | 0.58±0.06 (0.0064) |
| Glu450Ala | 0.085±0.03(0.009) | 120± 20(0.014) | 1.9±0.7 (0.02) | 3.4 ±0.18 (0.037) |

♥($x\ 10^{-4}.M^{-1}.min^{-1}$) §($x\ 10^{3}.min^{-1}$)

Notes on Table 2:

(a) The correlation coefficients of the corresponding linear plots were ≥0.95.
(b) The stated values represent the mean of at least three independent experiments.
(c) Numbers in parenthesis represent the values relative to the wild type.
(d) Concentration range of DFP: for wild type, Phe338Ala and Tyr337Ala 5 x $10^{-7}$M to 3 x $10^{-6}$M and for Glu202Gln and Glu450Ala 5 x $10^{-6}$M to 7.5 x $10^{-5}$M.
(e) Concentration range of soman**:for wild type, Phe338Ala and Tyr337Ala 4 x $10^{-10}$M to 1.4 x $10^{-8}$M and for Glu202Gln and Glu450Ala 1.4 x $10^{-8}$M to 2.8 x $10^{-7}$M.
(f) Concentration range of paraoxon: for wild type 1 x $10^{-8}$M to 1 x $10^{-7}$M, for Phe338Ala 1 x $10^{-7}$M to 2.5 x $10^{-6}$M, for Tyr337Ala 1 x $10^{-7}$M to 7.5 x $10^{-7}$M and for Glu202Gln and Glu450Ala 5 x $10^{-7}$M to 5 x $10^{-6}$M.
(g) All enzymes were 95-98% inhibited by soman*** and reactivated at various times by HI-6 (1 to 5 x $10^{-4}$M).

While presently preferred modes of operating the invention have been particularly described herein, it will be appreciated by those skilled in the art that many modifications and variations are possible. The invention is therefore not to be construed as limited by the modes of operation particularly described, but may be practised according to its concept, spirit or scope, as may be more readily understood by consideration of the appended claims.

## Claims

**1.** Use of at least one recombinant cholinesterase mutant in a method for the manufacture of a medicament for the detoxification of humans who have been exposed to a toxic phosphylating agent, by administration thereto so as to form phosphylated cholinesterase mutant, in presence of reactivator activity provided either by intrinsic reactivator activity in said mutant or by at least one discrete reactivator compound, said reactivator activity being effective to reactivate said mutant by breaking the phosphyl/cholinesterase mutant covalent bond, and wherein the at least one recombinant cholinesterase mutant is characterized by the fact that said thus-formed mutant phosphylated derivative is more readily reconverted thereto, in presence of said reactivator activity, than the corresponding phosphylated non-mutated cholinesterase is similarly reconverted to non-phosphylated non-mutated cholinesterase; the mutation(s) in the cholinesterase having been effected, for example, by replacing at least one amino acid at the positions indicated below in the amino acid sequence of HuAChE, or in equivalent positions in other cholinesterases, by a different amino acid, said at least one amino acid being replaced being selected from at least one of the following sub-groups (a), (b), (c) and (d):

(a) Ser2 0 3, Glu334, His447;

(b) Gly122, Tyr133, Glu2 0 2, Ser229, Gly448, Glu45 0 ;

(c) Leu76, Tyr77, Trp86, Tyr117, Tyr119, Gly12 0 , Gly121, Trp236, Phe295, Phe297, Tyr337, Phe338, Trp439, Tyr449;

(d) Tyr72, Asp74, Tyr124, Glu285, Trp286, Tyr341.

**2.** Use according to claim 1, wherein at least one of the following three conditions apply:

said phosphylating agent is selected from organophosphorus insecticides and organophosphorus nerve gases;

said medicament is a pharmaceutical formulation which comprises additionally at least one non-active ingredient selected from the group consisting of pharmaceutically acceptable carriers, diluents and adjuvants;

said medicament is adapted for oral, parenteral, percutaneous, rectal or topical administration.

**3.** A method for decontamination of a locus containing a toxic phosphylating agent, which comprises applying to the locus at least one recombinant cholinesterase mutant so as to form phosphylated cholinesterase mutant, in presence of reactivator activity provided either by intrinsic reactivator activity in said mutant or by at least one discrete reactivator compound, said reactivator activity being effective to reactivate said mutant by breaking the phosphyl/cholinesterase mutant covalent bond, and wherein the at least one recombinant cholinesterase mutant is characterized by the fact that said thus-formed mutant phosphylated derivative is more readily reconverted thereto, in presence of said reactivator activity, than the corresponding phosphylated non-mutated cholinesterase is similarly reconverted to non-phosphylated non-mutated cholinesterase; the mutation(s) in the cholinesterase having been effected, for example, by replacing at least one amino acid at the positions indicated below in the amino acid sequence of HuAChE, or in equivalent positions in other cholinesterases, by a different amino acid, said at least one amino acid being replaced being selected from at least one of the following sub-groups (a), (b), (c) and (d):

(a) Ser2 0 3, Glu334, His447;

(b) Gly122, Tyr133, Glu2 0 2, Ser229, Gly448, Glu45 0 ;

(c) Leu76, Tyr77, Trp86, Tyr117, Tyr119, Gly12 0 , Gly121, Trp236, Phe295, Phe297, Tyr337, Phe338, Trp439, Tyr449;

(d) Tyr72, Asp74, Tyr124, Glu285, Trp286, Tyr341.

**4.** Method according to claim 3, wherein at least one of the following two conditions apply:

said phosphylating agent is selected from organophosphorus insecticides and organophosphorus nerve gases;

said mutant is applied in the form of a composition which comprises additionally at least one non-active ingredient selected from the group consisting of diluents, carriers and surface active agents.

**5.** A recombinant cholinesterase mutant characterized by the facts that:

(i) a phosphylated derivative thereof is more readily reconverted to non-phosphylated mutant, than the corresponding phosphylated non-mutated cholinesterase is reconverted to nonphosphylated non-mutated cholinesterase; and

(ii) it comprises intrinsic reactivator activity effective to reactivate said mutant from a phosphylated product thereof, by breaking the phosphyl/cholinesterase mutant covalent bond;

the mutation(s) in the cholinesterase having been effected, for example, by replacing at least one amino acid at the positions indicated below in the amino acid sequence of HuAChE, or in equivalent positions in other cholinesterases, by a different amino acid, said at least one amino acid being replaced being selected from at least one of the following sub-groups (a), (b), (c) and (d):

(a) Ser2 0 3, Glu334, His447;

(b) Gly122, Tyr133, Glu2 0 2, Ser229, Gly448, Glu45 0 ;

(c) Leu76, Tyr77, Trp86, Tyr117, Tyr119, Gly12 0 , Gly121, Trp236, Phe295, Phe297, Tyr337, Phe338, Trp439, Tyr449;

(d) Tyr72, Asp74, Tyr1214, Glu285, Trp286, Tyr341.

**6.** A pharmaceutical formulation useful for detoxification of humans who have been exposed to a toxic phosphylating agent, which comprises a recombinant cholinesterase mutant as defined in claim 1 and reactivator activity provided either by intrinsic reactivator activity in said mutant or by at least one discrete reactivator compound in said formulation, said reactivator activity being effective to reactivate said mutant by breaking the phosphyl/cholinesterase mutant covalent bond; together with at least one non-active ingredient selected from the group consisting of pharmaceutically acceptable carriers, diluents and adjuvants, said formulation being preferably adapted for oral, parenteral, percutaneous, rectal or topical administration.

**7.** A composition useful for decontamination of a locus containing a toxic phosphylating agent, which comprises a recombinant cholinesterase mutant as defined in claim 1 and reactivator activity provided either by intrinsic reactivator activity in said mutant or by at least one discrete reactivator compound in said composition, said reactivator activity being effective to reactivate said mutant by breaking the phosphyl/cholinesterase mutant covalent bond; together with at least one non-active ingredient selected from the group consisting of diluents, carriers and surface active agents.

**8.** A phosphylated reaction product of a recombinant cholinesterase mutant with a tetravalent phosphorus electrophilic group, such phosphylated reaction product being characterized by the fact that it is more readily reconverted to non-phosphylated mutant, than the corresponding phosphylated non-mutated cholinesterase is reconverted to non-phosphylated non-mutated cholinesterase; provided that said phosphylated reaction product does not include the following categories (i) and (ii):

(i) Glu199Gln TcAChE and Glu199Asp TcAChE which have been phosphylated by reaction with a phosphylating agent selected from the group consisting of haloxon, paraoxon, DFP and MEPQ;

(ii) mouse AChE mutants Phe295Leu, Phe297Ile, Phe295Leu/Phe297Ile, Arg296Ser and Val300 Gly which have been phosphylated by reaction with $_2(Me_2CHNH)$ (O=)P-O-P(=O) $(NHCHMe_2)_2$

**9.** A phosphylated reaction product according to claim 8, wherein the mutation(s) in the cholinesterase has-(have) been effected by replacing at least one amino acid at the positions indicated below in the amino acid sequence of HuAChE, or in equivalent positions in other cholinesterases, by a different amino acid, said at least one amino acid being replaced being selected from at least one of the following sub-groups (a), (b), (c) and (d):

(a) Ser203, Glu334, His447;

(b) Gly122, Tyr133, Glu202, Ser229, Gly448, Glu450;

(c) Leu76, Tyr77, Trp86, Tyr117, Tyr119, Gly120, Gly121, Trp236, Phe295, Phe297, Tyr337, Phe338, Trp439, Tyr449;

(d) Tyr72, Asp74, Tyr124, Glu285, Trp286, Tyr341.

**10.** A phosphylated reaction product according to either claim 8 or claim 9, wherein said tetravalent phosphorus electrophilic group is derived from a compound of the formula

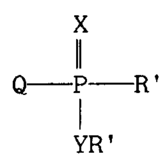

wherein Q is a leaving group, R' is R" or ZR", each of R' and R", which may be the same as or different from each other, is selected from the group consisting of $C_1$-$C_{10}$ alkyl, $C_3$-$C_{10}$ cycloalkyl and $C_3$-$C_{10}$ cycloalkyl substituted by up to two $C_1$-$C_4$ alkyl groups, and X, Y and Z are each independently oxygen or sulfur.

FIGURE 1

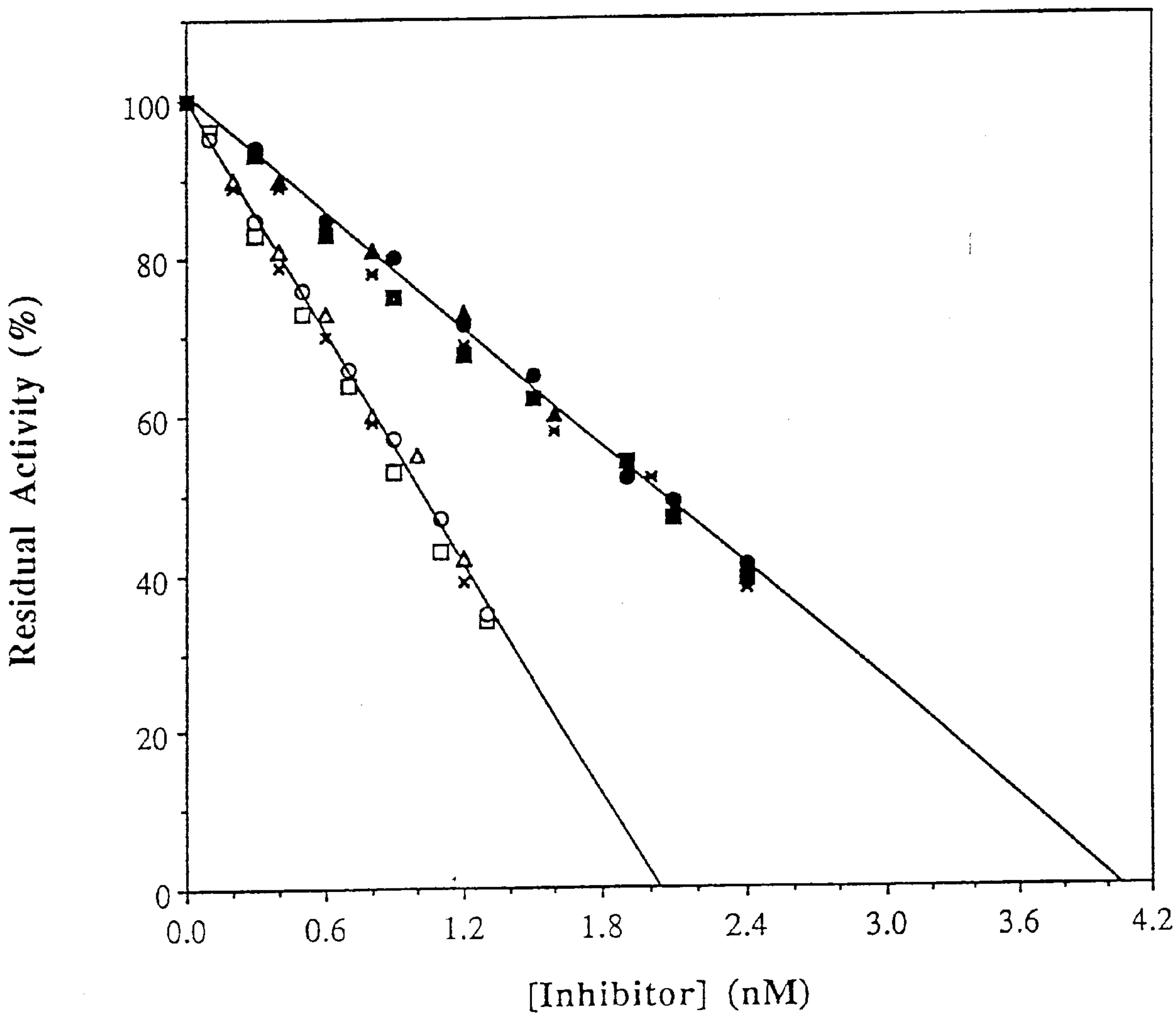
Residual Activity (%)
100
80
60
40
20
0
0.0
0.6
1.2
1.8
2.4
3.0
3.6
4.2
[Inhibitor] (nM)